# EUROPEAN PATENT APPLICATION

(11) **EP 0 716 844 A1**
(43) Date of publication of application: **19.06.1996**
(21) Application number: 95307914.2
(22) Date of filing: 06.11.1995
(51) Int. Cl.: A61H 33/02

(54) **Apparatus for treating patients with oxygen**

(30) Priority: 13.12.1994 GB 9425072
(71) Applicant: The BOC Group plc, Windlesham Surrey GU20 6HJ (GB)
(72) Inventor: Garrett, Michael Ernest, Woking, Surrey GU22 7XR (GB)
(74) Representative: Gough, Peter

(57) **Abstract**

An apparatus 1 for the ectopic treatment of a patient with oxygen comprises a circuit including a bath 2 for receiving the patient, a container 6 spaced from the bath 2 and containing the oxygenated liquid and a pump 12 for circulating the oxygenated liquid between the container 6 and the bath 2.

Preferably, the bath is located in a hyperbaric chamber 4.

## Description

The present invention relates to apparatus for the ectopic treatment of patients with oxygen.

It is known to place a patient in a chamber containing oxygen at high pressures to undergo radio therapy and various treatments of for example; certain forms of poisoning, bacterial infections and poor healing wounds. Hyperbaric Oxygen up to 2 bara pressure is often administered to patients by enclosing them in a pressure chamber which is pressurised with air and then providing breathing oxygen through a mask. The treatment has been shown to be effective with a number of illnesses but its healing effect is limited to those tissues in direct contact with the oxygen. Furthermore, although the partial pressure of oxygen in the pressurised air surrounding the patient is increased relative to atmosphere it is still relatively low.

It is an aim of the present invention to provide an apparatus which will enable a patient to experience oxygen enhancement of up to 1 bar g partial pressure in the breathing gas while at the same time the cutaneous tissues of the patient can be exposed to oxygen partial pressures of 5 bar g. It is believed that this is particularly effective when bacterial infections of the skin are involved.

According to the present invention, an apparatus for the ectopic treatment of a patient with oxygen comprises a bath for receiving the patient and is characterised by a source of oxygenated liquid spaced from the bath at which oxygen is dissolved and remains dissolved in the liquid and means for delivering the oxygenated liquid to the bath.

Preferably, the oxygenated liquid is oxygenated water and the means includes a pump for circulating the oxygenated water between said source and the bath.

In a preferred embodiment the bath is located in a hyperbaric chamber.

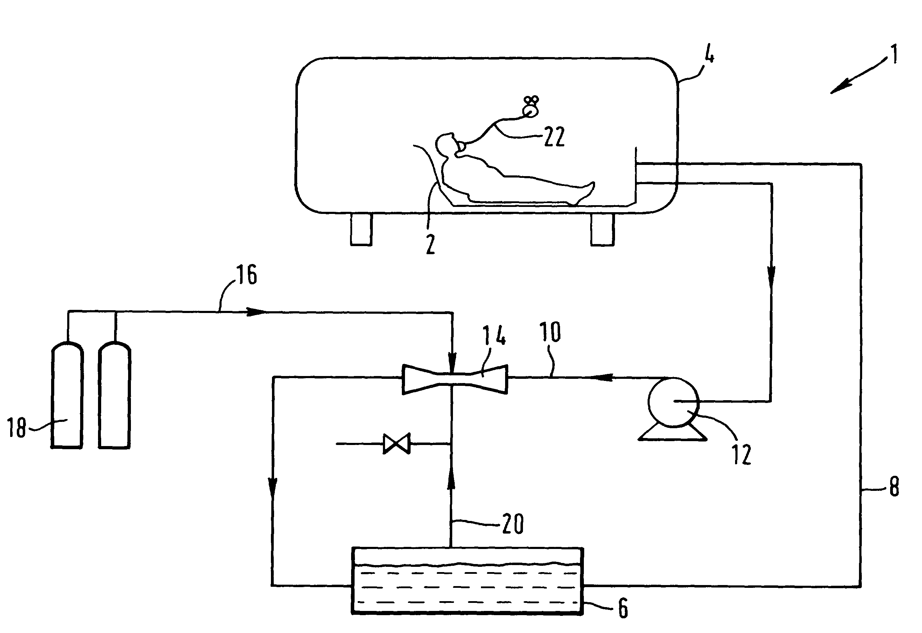

An embodiment of the invention will now be described by way of example, reference being made to the Figure of the accompanying diagrammatic drawing which is a diagrammatic sketch of an apparatus for the ectopic treatment of patients with oxygen.

As shown, an apparatus 1 for exposing the skin of a patient to contact with an oxygenated liquid such as water includes a bath 2 for the patient positioned within a hyperbaric chamber 4. The bath 2 is located in a circuit which includes a source of oxygenated water including a container 6 spaced from the bath 2, a first pipeline 8 from the container 6 to the bath 2 and a second pipeline 10 from the bath 2 to the container 6 in which is located both a pump 12 and a venturi 14.

A third pipeline 16 extends from a source 18 of gaseous oxygen under pressure towards the venturi 14 and a fourth pipeline 20 extends from the upper (as shown) surface of the container 6 again towards the venturi 14.

In use, a patient is placed in the bath 2 and is supported by water saturated with oxygen. The water may be at body temperature and is gently circulated by the pump 12. The water leaving the bath 2 through the pipeline 10 passes through the venturi 14 where it is re-oxygenated by the passage of fresh oxygen from the source 18 passing through the pipeline 16. Any excess oxygen in the ullage space above the water level in the container 6 passes through the pipeline 20 towards the venturi 14.

The oxygen dissolved in the water contained within container 6 will remain dissolved during its delivery to and whilst it remains in the bath 2.

In this embodiment, where the bath 2 is positioned in the hyperbaric chamber 4, the chamber 4 can be pressurised by means known per se to approximately 5 bar a and the patient can wear a breathing mask which will include a tube 22 for the passage therethrough of a breathable gas e.g air, at a pressure of approximately 5 bar a.

It will be apparent that the patient can be surrounded by oxygen at partial pressures up to 5 barg, the composition of the breathing gas being adjusted to accommodate the external pressure. Furthermore, the apparatus avoids the risk of fire because of the absence of an oxygen enriched gas.

The atmosphere above the bath and within the hyperbaric chamber can also be monitored for oxygen enrichment and can be replaced by air or partly replaced by nitrogen at the same pressure.

The gas breathed by the patient can be a mixture such as oxygen/helium which avoids the increased density of the pressurised gas and is then far easier to breathe. Replacing nitrogen with helium also reduces the risk of nitrogen dissolving in the blood during the prolonged periods of treatment.

To reiterate the apparatus described above avoids any fire risk and at the same time allows higher partial pressures of oxygen to be used externally of the body of the patient.

## Claims

1. An apparatus 1 for the ectopic treatment of a patient with oxygen comprising a bath 2 for receiving the patient is characterised by a source of oxygenated liquid spaced from the bath 2 at which oxygen is dissolved and remains dissolved in the liquid and means for delivering the oxygenated liquid to the bath 2.

2. An apparatus as claimed in Claim 1, in which the delivery means includes a pump 12 for circulating the oxygenated liquid between said source and the bath 2.

3. An apparatus as claimed in Claim 1 or 2, in which the source includes a container 6 for the oxygenated liquid to which is connected a source 18 of gaseous oxygen.

4. An apparatus as claimed in Claim 3, in which a venturi 14 is located between the source 18 of gaseous oxygen and the container 6 for oxygenated liquid.

5. An apparatus as claimed in any one of Claims 1 to 4, in which the bath 2 is located in a hyperbaric chamber 4.

6. An apparatus as claimed in Claim 5, in which a tube 22 for the passage therethrough of a breathable gas extends into the hyperbaric chamber 4 for use by a patient when received within the bath 2.

7. An apparatus as claimed in any one of Claims 1 to 6, in which the oxygenated liquid is oxygenated water.

8. An apparatus for the ectopic treatment of patients with oxygen constructed, arranged and adapted to operate substantially as herein before described with reference to the Figure of the accompanying drawing.
